# DEMANDE DE BREVET EUROPEEN

(11) **EP 1 108 414 A1**
(43) Date de publication de la demande: **20.06.2001**
(21) Numéro de dépôt: 00403472.4
(22) Date de dépôt: 11.12.2000
(51) Int. Cl.: A61K 7/02, C04B 28/14

(54) **Composition cosmétique solide à base de plâtre comprenant un agent modificateur de la cinétique de prise particulier, procédé de préparation et utilisations**

(30) Priorité: 15.12.1999 FR 9915818
(71) Demandeur: L'OREAL, 75008 Paris (FR)
(72) Inventeur: Robert, Valérie, 75003 Paris (FR); Lemann, Patricia, 94000 Creteil (FR); Collette, Annick, 94100 Saint-Maur des Fosses (FR)
(74) Mandataire: Doressamy, Clarisse

(57) **Abrégé**

L'invention concerne une composition cosmétique solide à base de plâtre comprenant un agent modificateur de la cinétique de prise particulier choisi parmi les monophosphates de calcium, les condensats formaldéhyde/acide 4-aminobutyrique/sel de calcium, les gypses enrobés d'amidon et leurs mélanges, utilisable en particulier dans le domaine du maquillage. Elle concerne également un procédé de préparation d'une composition, notamment cosmétique, solide à base de plâtre comprenant les étapes suivantes :
- a°) on prépare un mélange pulvérulent comprenant au moins du sulfate de calcium hémihydraté sous forme de poudre,
- b°) on ajoute au mélange pulvérulent un phase aqueuse,
- c°) on malaxe le mélange pulvérulent et la phase aqueuse de façon à obtenir un mélange coulable,
- d°) on verse le mélange coulable dans un moule,
- e°) on laisse durcir le mélange par hydratation du sulfate de calcium hémihydraté en sulfate de calcium dihydraté,
caractérisé par le fait qu'il comprend en outre une étape d'introduction d'un agent modificateur de la cinétique de prise particulier.

L'invention concerne aussi une composition, notamment cosmétique, susceptible d'être obtenue par le procédé.

## Description

La présente invention concerne une composition cosmétique solide à base de plâtre utilisable en particulier dans le domaine du maquillage. Elle concerne également un procédé de fabrication d'une composition solide à l'aide de plâtre et une composition, notamment cosmétique, obtenue par ce procédé.

Il est connu de fabriquer des compositions cosmétiques sous forme solide à l'aide de plâtre. Ces compositions cosmétiques peuvent être notamment des fards à paupières, des fards à joues, des poudres pour le visage ou pour le corps. Elles peuvent se présenter sous forme de stick, de crayons ou de pains. L'utilisation du plâtre pour la fabrication de ces compositions solides est, par exemple, décrit dans le brevet US-A-4724138. Selon les procédés connus, une phase pulvérulente contenant du plâtre est mélangée avec une phase aqueuse pour obtenir une pâte que l'on met en forme par moulage, la réaction d'hydratation du plâtre (sulfate de calcium hémihydraté) en sulfate de calcium dihydraté provoquant la solidification de la composition. L'utilisation de plâtre comme agent de solidification est avantageuse car elle permet de remplacer l'opération de compactage, habituellement nécessaire pour obtenir une composition sous forme solide à partir de produits pulvérulents, par une opération de moulage, dont la mise en oeuvre est plus simple, moins coûteuse et permet d'obtenir des formes plus diverses.

La demanderesse a constaté que ces compositions n'étaient pas encore tout à fait satisfaisantes en ce qui concerne la cohésion et la résistance du produit final : en particulier, les compositions peuvent adhérer à leur article de conditionnement, elles peuvent rester accrochées à cet article de conditionnement. Les compositions présentent alors des lacunes, des trous, leur aspect extérieur n'est plus uniforme. Le produit fini manque de résistance. Il peut ainsi s'avérer qu'à l'ouverture du produit, une partie de la composition se détache et soit perdue pour la consommatrice. Ce phénomène peut en particulier être observé lorsque la composition comprend par exemple un fort taux de particules de grande taille ou encore lorsqu'elle comprend diverses charges dont les formes des particules sont très variées.

La présente invention a pour but de pallier ces inconvénients.

La demanderesse a découvert de façon inattendue et surprenante que l'ajout d'un composé particulier, à savoir un agent modificateur de la cinétique de prise, au sein de ces compositions, permettait de réaliser des compositions présentant un aspect extérieur lisse et uniforme.

Comme expliqué ci-dessus, pour réaliser une composition à base de plâtre, on prépare de façon classique un mélange pulvérulent comprenant du sulfate de calcium hémihydraté qu'on mélange ensuite avec de l'eau pour convertir ce sulfate de calcium hémihydraté en sulfate de calcium dihydraté, aboutissant ainsi à un produit solide. On appelle temps de prise le temps que met la pâte « mélange pulvérulent + eau » pour devenir solide.

Il est connu dans l'industrie de fabrication du plâtre d'utiliser des agents modificateurs du temps de prise dans les compositions à base de plâtre, en particulier pour des raisons de contraintes industrielles (existence d'un temps de prise cible, temps nécessaire pour les opérations de coulage, etc...).

Toutefois, il n'a jamais été décrit qu'un agent modificateur de la cinétique de prise particulier pouvait permettre de réaliser une composition cosmétique à base de plâtre présentant une surface particulièrement lisse et uniforme.

Il a en particulier été observé que les agents modificateurs de la cinétique de prise tels que les monophosphates de calcium, les condensats formaldéhyde/acide 4-aminobutyrique/sel de calcium, éventuellement dispersés dans du sulfate de calcium, les gypses enrobés d'amidon et/ou leurs mélanges permettaient d'obtenir des compositions dont la surface est particulièrement homogène.

La présente invention a donc pour objet l'utilisation d'un agent modificateur de la cinétique de prise choisi parmi les monophosphates de calcium, les condensats formaldéhyde/acide 4-aminobutyrique/sel de calcium, éventuellement dispersés dans du sulfate de calcium, les gypses enrobés d'amidon et leurs mélanges, dans ou pour la préparation d'une composition cosmétique solide comprenant du sulfate de calcium dihydraté, dans le but de conférer à ladite composition une surface lisse et uniforme.

La présente invention a donc également pour objet une composition cosmétique solide comprenant du sulfate de calcium dihydraté, caractérisée en ce qu'elle comprend en outre un agent modificateur de la cinétique de prise choisi parmi les monophosphates de calcium, les condensats formaldéhyde/acide 4-aminobutyrique/sel de calcium, éventuellement dispersés dans du sulfate de calcium, les gypses enrobés d'amidon et/ou leurs mélanges, et un milieu cosmétiquement et/ou physiologiquement acceptable.

Elle a également pour objet un procédé de préparation d'une composition cosmétique solide à base de plâtre comprenant les étapes suivantes :
- a°) on prépare un mélange pulvérulent comprenant au moins du sulfate de calcium hémihydraté sous forme de poudre,
- b°) on ajoute au mélange pulvérulent une phase aqueuse,
- c°) on malaxe le mélange pulvérulent et la phase aqueuse de façon à obtenir un mélange coulable,
- d°) on verse le mélange coulable dans un moule,
- e°) on laisse durcir le mélange par hydratation du sulfate de calcium hémihydraté en sulfate de calcium dihydraté,
   caractérisé par le fait qu'il comprend en outre une étape d'introduction d'un agent modificateur de la cinétique de prise choisi parmi les monophosphates de calcium, les condensats formaldehyde/acide 4-aminobutyrique/sel de calcium, éventuellement dispersés dans du sulfate de calcium, les gypses enrobés d'amidon et leurs mélanges.

L'invention concerne aussi une composition cosmétique susceptible d'être obtenue par le procédé selon l'invention. La composition ainsi obtenue convient parfaitement pour le maquillage. Elle peut être utilisée comme fard à joue, fard à paupières, ou poudre pour le visage et/ou pour le corps.

Les compositions obtenues ne présentent pas de lacunes, d'aspérités ou d'agglomérats. Elles n'adhèrent pas à leur conditionnement quel qu'il soit. Elles présentent une bonne cohésion, une bonne résistance et un aspect extérieur particulièrement uniforme et lisse.

La présente invention a également pour objet un procédé non thérapeutique de maquillage de la peau, caractérisé par le fait qu'on applique sur la peau une composition telle que définie ci-dessus.

L'agent modificateur de la cinétique de prise utilisable selon l'invention peut être choisi parmi les agents retardateurs du temps de prise comme le citrate de sodium, le monophosphate de calcium, les condensats formaldéhyde/acide 4-aminobutyrique/sel de calcium, éventuellement dispersés dans du sulfate de calcium, les agents accélérateurs du temps de prise comme le gypse, le gypse enrobé d'amidon, les sulfates de sodium, et leurs mélanges.

De préférence, l'agent modificateur de la cinétique de prise est choisi parmi les monophosphates de calcium, les condensats formaldéhyde/acide 4-aminobutyrique/sel de calcium dispersés dans du sulfate de calcium, le gypse enrobé d'amidon et/ou leurs mélanges.

A titre d'exemples de produits commerciaux, on peut citer le tétra-hydrogénophosphate de calcium monohydraté, vendu sous la dénomination commerciale « Retardateur RA 01 » par la société Lafarge, le condensat formaldéhyde/acide 4-aminobutyrique/sel de calcium dispersé à 11% dans le sulfate de calcium vendu sous la dénomination commerciale « Retardan GK » par Tricosal, le gypse enrobé d'amidon vendu sous la dénomination commerciale « BMA » par Prestia Lafarge.

Dans une forme préférée de réalisation de l'invention, l'agent modificateur de la cinétique de prise comprend un condensat formaldéhyde/acide 4-aminobutyrique/sel de calcium dispersé dans du sulfate de calcium. De préférence encore, cet agent comprend un mélange de i) condensat formaldéhyde/acide 4-aminobutyrique/sel de calcium dispersé dans du sulfate de calcium et de ii) gypse enrobé d'amidon. De préférence, le condensat formaldéhyde/acide 4-aminobutyrique/sel de calcium est dispersé à 11% dans le sulfate de calcium.

Dans une autre forme préférée de réalisation de l'invention, l'agent modificateur de la cinétique de prise comprend un monophosphate de calcium.

De préférence, l'agent modificateur de la cinétique de prise selon l'invention comprend en outre du citrate de sodium.

L'étape d'introduction de l'agent modificateur selon l'invention peut être réalisée à tout moment du procédé de fabrication des compositions selon l'invention, en particulier lors de l'étape a°) de préparation du mélange pulvérulent ou encore en même temps que l'étape b°) d'ajout de la phase aqueuse. L'agent modificateur de la cinétique de prise peut être sous forme d'un composé pulvérulent ou dissous dans un solvant.

De préférence, l'agent modificateur selon l'invention est ajouté dans le mélange pulvérulent, au cours de l'étape a°) du procédé.

L'agent modificateur de cinétique de prise est généralement présent dans le mélange pulvérulent à une teneur allant de 0,001 à 5%, de préférence de 0,01 à 1%, et de préférence encore de 0,01 à 0,6 %, en poids, par rapport au poids du mélange pulvérulent.

Il ressort du procédé de fabrication des compositions selon l'invention que la teneur en poids, par rapport au poids total du mélange pulvérulent préparé à l'étape a°) de ce procédé, d'un composé pulvérulent particulier, est sensiblement égale à la teneur en poids, par rapport au poids total de la composition finale, dudit composé pulvérulent particulier.

Ainsi, l'agent modificateur de cinétique de prise est généralement présent dans les compositions finales selon l'invention à une teneur pouvant aller de 0,001 à 5%, de préférence de 0,01 à 1% en poids, et de préférence encore à une teneur allant de 0,01 à 0,6% en poids, par rapport au poids total de la composition finale.

Le sulfate de calcium hémihydraté utilisé selon l'invention peut être sous sa forme a et/ou sous sa forme β. Le sulfate de calcium hémihydraté est un composé bien connu de l'homme du métier.

La quantité de plâtre (sulfate de calcium hémihydraté) présent dans le mélange pulvérulent peut aller de 10 % à 70 % en poids par rapport au poids total du mélange pulvérulent, de préférence de 15 % à 35 % et plus préférentiellement de 20 % à 30%.

Ainsi, le sulfate de calcium dihydraté est présent dans la composition finale à une teneur allant généralement de 10 à 70% en poids, de préférence de 15 à 35% en poids, par rapport au poids de la composition finale.

Par milieu cosmétiquement et/ou physiologiquement acceptable, on entend, au sens de la présente invention un milieu compatible avec la peau, autrement dit toute zone cutanée du corps et du visage.

La composition selon l'invention peut avantageusement comprendre en outre au moins une matière pulvérulente hydrophile.

Par matière pulvérulente hydrophile, on entend aussi bien des matières pulvérulentes hydrophiles par nature (non traitées par enrobage ou greffage chimique), que des matières pulvérulentes traitées par enrobage ou greffage chimique de façon à lui donner des caractéristiques hydrophiles.

Pour déterminer si, selon l'invention, une matière pulvérulente est hydrophobe ou hydrophile, on effectue le test ci-après défini. On remplit avec 20 ml d'eau un tube à essai d'un diamètre de 20 mm. On verse 2 grammes de poudre dans le tube sans agiter et on observe le comportement de la poudre pendant 5 minutes au maximum. Si la poudre reste parfaitement en surface, elle est considérée comme "hydrophobe". Dans le cas contraire, elle est considérée comme "hydrophile".

Les matières pulvérulentes hydrophiles naturellement peuvent par exemple être choisies parmi :
- les micas, qui sont des silicates d'aluminium et de potassium de compositions variées, d'origine naturelle, tels que la muscovite, la phlogopite, la lépidolite, la biotite et la séricite, ou d'origine synthétique,
- l'oxychlorure de bismuth,
- les silices, qui peuvent être sous forme de plaquettes ou de sphères telles que la silice commercialisée sous la dénomination "SILICA BEADS SB 700" par la Société MIYOSHI,
- les poudres de polymères hydrophiles, qui sont d'origine synthétique comme les polyacrylates, par exemple celui commercialisé sous la dénomination "MICROPEARL M 100" par la Société MATSUMOTO, les polyamides acryliques tels que ceux commercialisés par la Société ORIS, les polyuréthannes tels que celui commercialisé sous la dénomination "PLASTIC POWDER D 800" par la Société TOSHNU, les dérivés de cellulose ou d'amidon, par exemple les microsphères poreuses de cellulose,
- le kaolin, qui est un silicate d'aluminium hydraté,
- l'hydroxyapatite,
- les oxydes de zinc ou de titane, pour leur couvrance notamment, ces produits pouvant être utilisés à l'état nanopigmentaire pour leur effet filtrant,
- le carbonate de calcium,
- les carbonates et hydrocarbonates de magnésium, qui facilitent la fixation des parfums,
- et/ou leurs mélanges.

Les matières pulvérulentes hydrophiles peuvent également être des matières pulvérulentes qui ont été rendus hydrophiles par enrobage ou greffage chimique à l'aide de matières telles que le chitosane, le dioxyde de titane, la silice ou des polymères hydrophiles, notamment des polyesters sulfoniques ou des polyammonium quaternaire, et/ou leurs mélanges.

Ainsi, les matières pulvérulentes hydrophiles peuvent être choisies parmi des talcs traités et/ou enrobés hydrophiles, des poudres de polyamide traitées et/ou enrobées hydrophiles, des poudres de polyéthylène traitées et/ou enrobées hydrophiles, des poudres de copolymère expansé de chlorure de vinylidène, d'acrylonitrile et de (méth)acrylate de méthyle traitées et/ou enrobées hydrophiles, des poudres polyfluorées traitées et/ou enrobées hydrophiles, des poudres de silicone traitées et/ou enrobées hydrophiles, des poudres de copolymère acrylique traitées et/ou enrobées hydrophiles, des poudres de polystyrène traitées et/ou enrobées hydrophiles, des pigments traités et/ou enrobés hydrophiles, et/ou leurs mélanges.

A titre d'exemples de matières pulvérulentes hydrophiles, on peut citer le talc enrobé de chitosane vendu par Daito sous le nom "Talc CT 2 MSA", le mica enrobé de microsphères de silice vendu par Catalysts et Chemicals sous le nom "Cashmir B3".

Les matières pulvérulentes hydrophiles peuvent également être choisies parmi les pigments et/ou les nacres et/ou les paillettes hydrophiles utilisables en cosmétique.

Parmi les pigments minéraux, on peut citer, à titre d'exemple :
- les oxydes de fer noir, jaune, rouge et brun, codifiés dans le Color Index sous les références Cl 77499, Cl 77492 et Cl 77491,
- le violet de manganèse (Cl 77742),
- le bleu d'outremer (Cl 77007),
- le violet d'outremer (Cl 77007),
- l'oxyde de chrome (Cl 77288),
- l'oxyde de chrome hydraté (Cl 77289) et
- le bleu ferrique (Cl 77510),
- et/ou leurs mélanges.

Parmi les pigments organiques, on peut citer, en particulier, les pigments, éventuellement sur des laques organiques :
- D & C red n° 3 (Cl 45430:1),
- D & C red n° 6 (Cl 15850:2),
- D & C red n° 7 (Cl 15850:1),
- D & C red n° 9(Cl 15585:1),
- D & C red n° 13 (Cl 15630:3),
- D & C red n° 19 (Cl 45170),
- D & C red n° 21 (Cl 45380 : 2),
- D & C red n° 27 (Cl 45410:1),
- D & C red n° 30 (Cl 73360),
- D & C red n° 36 (Cl 12085),
- le noir de carbone (Cl 77266) et les laques à base de carmin de cochenille (Cl 75470),
- D & C yellow n° 5 (Cl 19140),
- D & C red n° 40 (Cl 16035:1),
- FD & C blue n°1 (Cl 42090 :2)
- et/ou leurs mélanges.

Par nacres, il faut comprendre des particules irisées qui réfléchissent la lumière.

Les nacres peuvent être choisies parmi les pigments nacrés tels que le mica recouvert de pigments organiques et/ou minéraux tel que l'oxyde de titane ou l'oxychlorure de bismuth, le mica titane recouvert de pigments organiques et/ou minéraux tel que les oxydes de fer, le bleu ferrique ou l'oxyde de chrome, ainsi que les pigments nacrés à base d'oxychlorure de bismuth.

La quantité de matière pulvérulente hydrophile introduite dans le mélange pulvérulent peut aller de 5 % à 95 % en poids par rapport au poids total du mélange pulvérulent, et de préférence de 25 % à 80 %.

Ainsi, de préférence, les matières pulvérulentes hydrophiles sont présentes dans la composition finale à une teneur allant de 5 à 95%, en poids, de préférence encore de 25 à 80%, en poids, par rapport au poids total de la composition finale.

La composition selon l'invention peut également comprendre au moins une matière pulvérulente hydrophobe.

Les matières pulvérulentes hydrophobes peuvent être choisies parmi les matières pulvérulentes hydrophobes par nature comme par exemple :
- le talc qui est un silicate de magnésium hydraté,
- les poudres de polymères hydrophobes, telles que la poudre des polyamides de type nylon, par exemple celle commercialisée sous la dénomination "ORGASOL 2002 ED NAT COS" par la Société ATOCHEM, la poudre de polyéthylène, par exemple celle commercialisée sous la dénomination "COATHYLENE HA 1681" par la Société PLAST LABOR ; les microsphères expansées en matériau thermoplastique, par exemple celle commercialisée sous la dénomination "EXPANCEL 551 DE" par la Société CASCO-NOBEL, les poudres polyfluorées notamment de polytétrafluoroéthylène, par exemple celle commercialisée sous la dénomination "MP 1400" par la Société DUPONT DE NEMOURS, les poudres de silicone, par exemple celles commercialisées sous la dénomination "TOSPEARL" par la Société TOSHIBA, les poudres de copolymère acrylique, telles que celles commercialisées sous la dénomination "POLYTRAP Q5 6603" par la Société DOW CHEMICA, ou encore les poudres de polystyrène telles que celles commercialisées sous la dénomination "POLYSPHERE 3 000 SP" par la Société PRESPERESE ;
- les lipoaminoacides, par exemple la lauroyl lysine,
- le nitrure de bore,
- les savons métalliques d'acides carboxyliques en C₈-C₂₂, plus particulièrement en C₁₂-C₁₈, par exemple les stéarates de zinc et de magnésium, le laurate de zinc ou le myristate de magnésium,
- et/ou leurs mélanges.

Les matières pulvérulentes hydrophobes peuvent aussi être choisies parmi des matières pulvérulentes de nature aussi bien hydrophobe qu'hydrophile que l'on a rendues hydrophobes en les traitant par enrobage ou greffage chimique par des produits tels que les silicones, les aminoacides, les savons métalliques, les dérivés fluorés, les huiles minérales, la lécithine, le triisostéaroyl titanate d'isopropyle, le polyéthylène, le collagène et ses dérivés, les polyacrylates, et/ou leurs mélanges.

Ainsi, les matières pulvérulentes hydrophobes peuvent être choisies parmi des micas enrobés et/ou traités hydrophobes, des silices enrobées et/ou traitées hydrophobes, du kaolin enrobé et/ou traité hydrophobe, des oxydes métalliques enrobés et/ou traités hydrophobes comme les oxydes de titane enrobés et/ou traités hydrophobes, les oxydes de fer enrobés et/ou traités hydrophobes, les oxydes de zinc enrobés et/ou traités hydrophobes, et/ou leurs mélanges.

A titre d'exemples de matières pulvérulentes hydrophobes, on peut citer les microbilles de silice enrobées de polymethylhydrogenosiloxane vendues sous la dénomination commerciale "Silice SI SB 700" par Miyoshi ou encore la sericite enrobée de methicone/huile d'oeuf hydrogénée vendue sous la dénomination commerciale "Sericite SNI S100" par Miyoshi.

La quantité de matière pulvérulente hydrophobe introduite dans le mélange pulvérulent peut aller de 5 % à 60 % en poids par rapport au poids total du mélange pulvérulent, et de préférence de 10 % à 45 %.

Ainsi, de préférence, les matières pulvérulentes hydrophobes sont présentes dans la composition finale à une teneur allant de 5 à 60 %, en poids, de préférence encore de 10 à 45%, en poids, par rapport au poids total de la composition finale.

Avantageusement, le rapport pondéral de la quantité de matière pulvérulente hydrophile par rapport à la quantité de matière hydrophobe peut aller de 0,08 à 15, et de préférence de 0,40 à 8.

Dans une forme préférée de réalisation de l'invention, la composition finale comprend au moins un pigment, et de préférence encore, au moins deux pigments différents.

Ainsi, dans une forme préférée de réalisation de l'invention, les pigments sont présents à une teneur allant de 1 à 30%, en poids, de préférence de 10 à 20% en poids, par rapport au poids total de la composition finale.

Dans une forme préférée de réalisation de l'invention, la composition finale comprend au moins une nacre.

Ainsi, dans une autre forme préférée de réalisation de l'invention, les nacres sont présentes à une teneur allant de 1 à 50%, en poids, de préférence de 15 à 25% en poids, par rapport au poids total de la composition finale.

Les compositions de l'invention peuvent également comprendre des paillettes de polymères ou de copolymères de polyéthylène/téréphtalate ou de polyméthacrylate, éventuellement enrobés. Ces paillettes peuvent être présentes dans la composition selon l'invention à une teneur allant jusqu'à 10% en poids, par rapport au poids total de la composition.

Dans une forme préférée de réalisation de l'invention, la composition finale comprend au moins une matière pulvérulente de taille moyenne des particules supérieure ou égale à 100 nanomètres. De préférence, la matière pulvérulente de taille moyenne des particules supérieure ou égale à 100 nanomètres est présente dans le mélange pulvérulent à une teneur allant de 10 à 50 % en poids, de préférence encore de 15 à 35% en poids, par rapport au poids du mélange pulvérulent.

Ainsi, de préférence, la matière pulvérulente de taille moyenne des particules supérieure ou égale à 100 nanomètres est présente dans la composition finale à une teneur allant de 10 à 50%, en poids et de préférence encore de 15 à 35% en poids, par rapport au poids total de la composition finale.

Pour assurer une bonne cohésion à la composition finale, on peut ajouter à la phase pulvérulente au moins un corps gras. Comme corps gras utilisables, on peut citer les huiles minérales telles que l'huile de vaseline, les huiles d'origine animale telles que la lanoline, les huiles d'origine végétale telles que l'huile de jojoba, les esters d'acides carboxyliques et d'alcool gras en C10-C22, les esters d'acides gras et d'alcool tel que le palmitate d'isopropyle, les alcools gras, notamment les alcools gras en en C₁₀-C₂₂ tels que l'alcool oléylique, l'alcool isostéarylique, l'octyldodécanol, les huiles de synthèse telles que les poly alpha oléfines, hydrogénées ou non, comme le polyisobutène (Parléam), les polydécènes, les huiles de silicone, notamment les huiles de silicone phénylées, les gommes de silicone ou les cires de silicone telles que les alkyldiméthicones, les huiles fluorées.

De préférence, la quantité de corps gras ajouté dans le mélange pulvérulent peut aller de 0,1 % à 20 % en poids par rapport au poids total du mélange pulvérulent, et de préférence de 0,5 % à 15 %.

Ainsi, de préférence, les corps gras sont présents dans la composition finale à une teneur allant de 0,1 % à 20 % en poids, de préférence encore de 0,5 % à 15 %., en poids, par rapport au poids total de la composition finale.

Le mélange pulvérulent peut avantageusement comprendre au moins un agent tensioactif pour faciliter le mouillage et la dispersion du mélange pulvérulent. L'agent tensioactif peut être de nature non ionique comme les esters de sorbitane polyoxyéthylénés, de nature cationique comme les sels d'ammonium quaternaires, ou de nature amphotère comme les dérivés de bétaïne.

La quantité de tensioactif introduite dans le mélange pulvérulent peut aller de 0,1 % à 10 % en poids par rapport au poids total du mélange pulvérulent.

Ainsi, de préférence, le tensioactif est présent dans la composition finale à une teneur allant de 0,1 % à 10 % en poids, par rapport au poids total de la composition finale.

La phase aqueuse contient obligatoirement de l'eau en quantité suffisante pour hydrater le plâtre en sulfate de calcium dihydraté. Elle peut comprendre éventuellement au moins un additif soluble ou dispersible dans l'eau. Comme additifs on peut citer des polymères hydrosolubles ou hydrodispersibles, des tensioactifs, des cires. Des actifs cosmétiques peuvent aussi être ajoutés dans la phase aqueuse comme par exemple des agents hydratants tels que la glycérine, le propylène glycol, ou bien encore des agents antioxydants, des filtres solaires.

La phase aqueuse peut être mélangée au mélange pulvérulent dans une proportion pondérale mélange pulvérulent/phase aqueuse allant de 0,2 à 2 et de préférence de 0,5 à 1,5 de façon à obtenir un mélange coulable.

Il est également possible de rajouter à la composition un système conservateur qui peut être introduit soit dans la phase pulvérulente, soit dans la phase aqueuse. Ce système conservateur peut être sous forme liquide et introduit dans la composition comme décrit dans EP 864 322.

Lorsque la pâte obtenue par mélange du mélange pulvérulent et de la phase aqueuse est coulée dans un moule, on laisse durcir le mélange à température ambiante. Pour accélérer le temps de prise, il est possible de laisser sécher la pâte en plaçant les moules dans une enceinte chauffée à une température pouvant aller par exemple jusqu'à 25 °C. On laisse ensuite la composition sécher, par exemple à une température de 55°C. Lorsque la composition finale est bien sèche, elle peut être directement utilisée dans son moule. On peut aussi démouler la composition et placer celle-ci dans un conditionnement approprié.

La composition selon l'invention peut comprendre en outre tout additif usuellement utilisé dans le domaine cosmétique, tels que des antioxydants, des filtres UV, des colorants, des parfums, des huiles essentielles, des conservateurs, des actifs cosmétiques, des hydratants, des vitamines, des sphingolipides, et/ou leurs mélanges.

Ces additifs peuvent être présents dans la composition à raison de 0-15% en poids, par rapport au poids total de la composition.

Bien entendu l'homme du métier veillera à choisir ce ou ces éventuels additifs, et/ou leur quantité, de manière telle que les propriétés avantageuses de la composition selon l'invention ne soient pas, ou substantiellement pas, altérées par l'adjonction envisagée.

Les compositions selon l'invention peuvent se présenter sous la forme d'un produit cosmétique et notamment sous la forme d'un produit de maquillage, en particulier un fard à joues, un fard à paupières, une poudre pour le visage, un blush, un fond de teint, un anticerne, un produit de maquillage pour le corps ou bien encore d'un produit de soin pour le corps et/ou le visage.

On va maintenant donner des exemples illustrant la présente invention sans toutefois la limiter.

### Exemple 1 : (invention)

On a préparé un mélange pulvérulent ayant la formulation suivante :

| | | |
|---|---|---|
| - Sulfate de calcium hémihydraté | | 25 g |
| - Poudres hydrophiles | | 51,1 g |
| - dont pigments (oxydes de fer de taille moyenne des particules supérieure ou égale à 100 nm) | 9 g | |
| - dont nacres (mica-titanes) | 30 g | |
| - Poudres hydrophobes | | 14,97 g |
| dont talc | 8,1 g | |
| - Corps gras | | 8 g |
| - Système conservateur | | 0,63 % |
| - condensat formaldéhyde/acide 4-aminobutyrique/ sel de calcium en dispersion à 11% dans le sulfate de calcium vendu sous la dénomination commerciale « Retardan GK » par Tricosal | | 0,3 g |

A ces 100 g de mélange pulvérulent, on a ajouté 72,41 g d'eau puis on a malaxé le mélange jusqu'à obtention d'une pâte coulable. Du citrate de sodium peut être additionné soit dans le mélange pulvérulent, soit dans la phase aqueuse, si névessaire. La pâte a ensuite été versée dans des coupelles puis séchée.

La composition finale obtenue a donc la formulation suivante :

| | | |
|---|---|---|
| - Sulfate de calcium dihydraté | | 28,33 % |
| - Citrate de sodium | | 0,239 % |
| - Poudres hydrophiles | | 48,832 % |
| - dont pigments (oxydes de fer) | 8,6 % | |
| - dont nacres (mica-titanes) | 28,668 % | |
| - Poudres hydrophobes | | 14,065 % |
| - dont talc | 7,74 % | |
| - Corps gras | | 7,645 % |
| - Système conservateur | | 0,602 % |
| - condensat formaldéhyde/acide 4-aminobutyrique/ sel de calcium en dispersion à 11 % dans le sulfate de calcium vendu sous la dénomination commerciale « Retardan GK » par Lafarge | | 0,287 % |

Le produit obtenu après séchage se présente sous forme de pain et se délite facilement à l'aide d'une houpette. Il ne se casse pas, ne présente pas de trous ou de lacune. L'aspect extérieur de la composition est lisse.

### Exemple 2 : (comparatif)

On a préparé une composition identique à celle de l'exemple 1 mais dans laquelle le condensat formaldéhyde/acide 4-aminobutyrique/sel de calcium en dispersion à 11% dans le sulfate de calcium a été remplacé par un agent modificateur du temps de prise classique, à savoir le citrate de sodium. La composition obtenue s'accroche au packaging, elle s'effrite, elle n'est pas acceptable.

### Exemple 3 (invention)

On a préparé un mélange pulvérulent ayant la formulation suivante :

| | | |
|---|---|---|
| - Sulfate de calcium hémihydraté | | 24,95 g |
| - Poudres hydrophiles | | 45,3 g |
| - dont pigments (bleu ferrique) | 2,5 g | |
| - dont nacres (mica-titanes) | 20 g | |
| - Poudres hydrophobes | | 23,07 g |
| - dont talc | 16,2 g | |
| - Corps gras | | 6 g |
| - Système conservateur | | 0,63 g |
| - tétra-hydrogénophosphate de calcium monohydraté vendu sous la dénomination commerciale « Retardateur RA 01 » par la société Lafarge | | 0,05 g |

A ces 100 g de mélange pulvérulent, on a ajouté 88,68 g d'eau puis on a malaxé le mélange jusqu'à obtention d'une pâte coulable. Du citrate de sodium peut être additionné soit dans le mélange pulvérulent, soit dans la phase aqueuse, si névessaire. La pâte a ensuite été versée dans des coupelles puis séchée.

La composition finale obtenue a donc la formulation suivante :

| | | |
|---|---|---|
| - Sulfate de calcium dihydraté | | 28,282 % |
| - citrate de sodium | | 0,238 % |
| - Poudres hydrophiles | | 43,051 % |
| - dont pigments (bleu ferrrique) | 2,389 % | |
| - dont nacres (mica-titanes) | 19,112 % | |
| - Poudres hydrophobes | | 22,046 % |
| - dont talc | 15,481 % | |
| - Corps gras | | 5,733 % |
| - Système conservateur | | 0,602 % |
| - tétra-hydrogénophosphate de calcium monohydraté vendu sous la dénomination commerciale « Retardateur RA 01 » par la société Lafarge | | 0,048 % |

Le produit obtenu après séchage se présente sous forme de pain et se délite facilement à l'aide d'une houpette. Il ne se casse pas, ne présente pas de trous ou de lacune. L'aspect extérieur de la composition est lisse.

### Exemple 4 : (comparatif)

On a préparé une composition identique à celle de l'exemple 3 mais dans laquelle le tétra-hydrogénophosphate de calcium monohydraté a été remplacé par un agent modificateur du temps de prise classique, à savoir le citrate de sodium. La composition obtenue s'accroche au packaging, elle s'effrite, elle n'est pas acceptable.

### Exemple 5 (invention)

On a préparé un mélange pulvérulent ayant la formulation suivante :

| | | |
|---|---|---|
| - Sulfate de calcium hémihydraté | | 24,95 g |
| - Poudres hydrophiles | | 45,3g |
| - dont pigments (bleu ferrique) | 2,5 g | |
| - dont nacres (mica-titanes) | 20 g | |
| - Poudres hydrophobes | | 22,8 g |
| - dont talc | 16,2g | |
| - Corps gras | | 6 g |
| - Système conservateur | | 0,63 g |
| - condensat formaldéhyde/acide 4-aminobutyrique/ sel de calcium en dispersion à 11 % dans le sulfate de calcium vendu sous la dénomination commerciale « Retardan GK » par Lafarge | | 0,28 % |
| - gypse enrobé d'amidon | | 0,04 % |

A ces 100 g de mélange pulvérulent, on a ajouté 88,68 g d'eau puis on a malaxé le mélange jusqu'à obtention d'une pâte coulable. Du citrate de sodium peut être additionné soit dans le mélange pulvérulent, soit dans la phase aqueuse, si névessaire. La pâte a ensuite été versée dans des coupelles puis séchée.

La composition finale obtenue a donc la formulation suivante :

| | | |
|---|---|---|
| - Sulfate de calcium dihydraté | | 28,282 % |
| - citrate de sodium | | 0,238 % |
| - Poudres hydrophiles | | 43,051 % |
| - dont pigments (bleu ferrrique) | 2,389 % | |
| - dont nacres (mica-titanes) | 19,112 % | |
| - Poudres hydrophobes | | 21,788 % |
| - dont talc | 15,481 % | |
| - Corps gras | | 5,733 % |
| - Système conservateur | | 0,602 % |
| - condensat formaldéhyde/acide 4-aminobutyrique/ sel de calcium en dispersion à 11 % dans le sulfate de calcium vendu sous la dénomination commerciale « Retardan GK » par Lafarge | | 0,268 % |
| - gypse enrobé d'amidon | | 0,038 % |

Le produit obtenu après séchage se présente sous forme de pain et se délite facilement à l'aide d'une houpette. Il ne se casse pas, ne présente pas de trous ou de lacune. L'aspect extérieur de la composition est lisse.

### Exemple 6 : (comparatif)

On a préparé une composition identique à celle de l'exemple 5 mais dans laquelle le condensat formaldéhyde/acide 4-aminobutyrique/sel de calcium en dispersion à 11% dans le sulfate de calcium et le gypse enrobé d'amidon ont été remplacés par un agent modificateur du temps de prise classique, à savoir le citrate de sodium. La composition obtenue s'accroche au packaging, elle s'effrite, elle n'est pas acceptable.

## Revendications

1. Composition cosmétique solide comprenant du sulfate de calcium dihydraté, caractérisée en ce qu'elle comprend en outre un agent modificateur de la cinétique de prise choisi parmi les monophosphates de calcium, les condensats formaldéhyde/acide 4-aminobutyrique/sel de calcium, éventuellement dispersés dans du sulfate de calcium, les gypses enrobés d'amidon et/ou leurs mélanges, et un milieu cosmétiquement et/ou physiologiquement acceptable.

2. Composition selon la revendication 1, caractérisée en ce que l'agent modificateur de la cinétique de prise comprend un condensat formaldéhyde/acide 4-aminobutyrique/sel de calcium dispersé dans du sulfate de calcium.

3. Composition selon la revendication 2, caractérisée en ce que l'agent modificateur de cinétique de prise comprend un mélange de i) condensat formaldéhyde/acide 4-aminobutyrique/sel de calcium dispersé dans du sulfate de calcium et de ii) gypse enrobé d'amidon.

4. Composition selon la revendication 1, caractérisée en ce que l'agent modificateur de la cinétique de prise comprend un monophosphate de calcium.

5. Composition selon l'une quelconque des revendications précédentes, caractérisée en ce que l'agent modificateur de la cinétique de prise comprend en outre du citrate de sodium.

6. Composition selon l'une quelconque des revendications précédentes, caractérisée en ce que l'agent modificateur de cinétique de prise est présent à une teneur pouvant aller de 0,001 à 5%, de préférence de 0,01 à 1% en poids, et de préférence encore à une teneur allant de 0,01 à 0,6% en poids, par rapport au poids total de la composition finale.

7. Composition selon l'une quelconque des revendications précédentes, caractérisée en ce que le sulfate de calcium dihydraté est présent à une teneur allant de 10 à 70% en poids, de préférence de 15 à 35% en poids, par rapport au poids de la composition finale.

8. Composition selon l'une quelconque des revendications précédentes, caractérisée en ce qu'elle comprend au moins une matière pulvérulente hydrophile.

9. Composition selon la revendication précédente, caractérisée en ce que les matières pulvérulentes hydrophiles sont présentes à une teneur allant de 5 à 95% en poids, de préférence encore de 25 à 80%, en poids, par rapport au poids total de la composition finale.

10. Composition selon l'une quelconque des revendications précédentes, caractérisée en ce qu'elle comprend au moins une matière pulvérulente hydrophobe.

11. Composition selon la revendication précédente, caractérisée en ce que les matières pulvérulentes hydrophobes sont présentes à une teneur allant de 5 % à 60 %, en poids, de préférence encore de 10 % à 45 %, en poids, par rapport au poids total de la composition finale.

12. Composition selon la revendication 10 ou 11, caractérisée en ce que le rapport pondéral de la quantité de matière pulvérulente hydrophile par rapport à la quantité de matière hydrophobe peut aller de 0,08 à 15, et de préférence de 0,40 à 8.

13. Composition selon l'une quelconque des revendications 8 à 12, caractérisée en ce qu'elle comprend au moins un pigment, et de préférence encore, au moins deux pigments différents.

14. Composition selon la revendication précédente, caractérisée en ce que les pigments sont présents à une teneur allant de 1 à 30%, en poids, de préférence de 10 à 20% en poids, par rapport au poids total de la composition finale.

15. Composition selon l'une quelconque des revendications 8 à 14, caractérisée en ce qu'elle comprend au moins une nacre.

16. Composition selon la revendication précédente, caractérisée en ce que les nacres sont présentes à une teneur allant de 1 à 50%, en poids, de préférence de 15 à 25% en poids, par rapport au poids total de la composition finale.

17. Composition selon l'une quelconque des revendications précédentes, caractérisée en ce qu'elle comprend au moins une matière pulvérulente de taille moyenne des particules supérieure ou égale à 100 nanomètres.

18. Composition selon la revendication précédente, caractérisée en ce que la matière pulvérulente de taille moyenne des particules supérieure ou égale à 100 nanomètres est présente dans la composition finale à une teneur allant de 10 à 50%, en poids et de préférence encore de 15 à 35% en poids, par rapport au poids total de la composition finale.

19. Composition selon l'une quelconque des revendications précédentes, caractérisée en ce qu'elle comprend en outre un corps gras.

20. Composition selon la revendication précédente, caractérisée en ce que le corps gras est présent à une teneur allant de 0,1 % à 20 % en poids, de préférence encore de 0,5 % à 15 %, en poids, par rapport au poids total de la composition finale.

21. Composition selon l'une quelconque des revendications précédentes, caractérisée en ce qu'elle comprend en outre un tensioactif.

22. Composition selon la revendication précédente, caractérisée en ce que le tensioactif est présent à une teneur allant de 0,1 % à 10 % en poids, par rapport au poids total de la composition finale.

23. Composition selon l'une quelconque des revendications précédentes, caractérisée en ce qu'elle se présente sous la forme d'un produit de maquillage, en particulier un fard à joues, un fard à paupières, une poudre pour le visage, un blush, un fond de teint, un anticerne, un produit de maquillage pour le corps ou bien encore d'un produit de soin pour le corps et/ou le visage.

24. Procédé de préparation d'une composition cosmétique solide à base de plâtre comprenant les étapes suivantes :
- a°) on prépare un mélange pulvérulent comprenant au moins du sulfate de calcium hémihydraté sous forme de poudre,
- b°) on ajoute au mélange pulvérulent une phase aqueuse,
- c°) on malaxe le mélange pulvérulent et la phase aqueuse de façon à obtenir un mélange coulable,
- d°) on verse le mélange coulable dans un moule,
- e°) on laisse durcir le mélange par hydratation du sulfate de calcium hémihydraté en sulfate de calcium dihydraté,
caractérisé par le fait qu'il comprend en outre une étape d'introduction d'un agent modificateur de la cinétique de prise choisi parmi les monophosphates de calcium, les condensats formaldehyde/acide 4-aminobutyrique/sel de calcium, éventuellement dispersés dans du sulfate de calcium, les gypses enrobés d'amidon et leurs mélanges.

25. Procédé selon la revendication précédente, caractérisé par le fait que l'agent modificateur de la cinétique de prise comprend un condensat formaldéhyde/acide 4-aminobutyrique/sel de calcium dispersé dans du sulfate de calcium.

26. Procédé selon la revendication 24, caractérisé en ce que l'agent modificateur de la cinétique de prise comprend un mélange de i) condensat formaldéhyde/acide 4-aminobutyrique/sel de calcium dispersé dans du sulfate de calcium et de ii) gypse enrobé d'amidon.

27. Procédé selon la revendication 24, caractérisé par le fait que l'agent modificateur de la cinétique de prise comprend un monophosphate de calcium.

28. Procédé selon l'une quelconque des revendications 24 à 27, caractérisé en ce que l'agent modificateur de la cinétique de prise comprend en outre du citrate de sodium.

29. Procédé selon l'une quelconque des revendications 24 à 28, caractérisé en ce que l'agent modificateur de la cinétique de prise est ajouté dans le mélange pulvérulent au cours de l'étape a°).

30. Procédé selon la revendication précédente, caractérisé en ce que l'agent modificateur de cinétique de prise est présent dans le mélange pulvérulent à une teneur allant de 0,001 à 5%, de préférence de 0,01 à 1 %, et de préférence encore de 0,01 à 0,6 %, en poids, par rapport au poids du mélange pulvérulent.

31. Procédé selon l'une quelconque des revendications 24 à 30, caractérisé par le fait que l'on malaxe le mélange pulvérulent et la phase aqueuse dans une proportion pondérale allant de 0,2 à 2, de préférence de 0,5 à 1,5.

32. Composition susceptible d'être obtenue par le procédé selon l'une quelconque des revendications 24 à 31.

33. Utilisation d'un agent modificateur de la cinétique de prise choisi parmi les monophosphates de calcium, les condensats formaldéhyde/acide 4-aminobutyrique/sel de calcium, éventuellement dispersés dans du sulfate de calcium, les gypses enrobés d'amidon et leurs mélanges, dans ou pour la préparation d'une composition cosmétique solide comprenant du sulfate de calcium dihydraté, dans le but de conférer à ladite composition une surface lisse et uniforme.

34. Procédé non thérapeutique de maquillage de la peau, caractérisé par le fait qu'on applique sur la peau une composition telle que définie à l'une quelconque des revendications 1 à 23 et 32.
